# EUROPEAN PATENT SPECIFICATION

(11) **EP 2 691 506 B1**
(45) Date of publication and mention of the grant of the patent: **23.08.2023**
(21) Application number: 12720971.6
(22) Date of filing: 28.03.2012
(51) Int. Cl.: C12M 1/107

(54) **AN APPARATUS FOR WASTE SEPARATION**
ABFALLTRENNUNGSVORRICHTUNG
APPAREIL POUR LA SÉPARATION DES DÉCHETS

(30) Priority: 28.03.2011 US 201161468117 P
(43) Date of publication of application: 05.02.2014
(73) Proprietor: Rainbowtec Environmental Solutions 2010 Ltd., 22825 Kfar Rosh Hanikra (IL)
(72) Inventor: ZAID, Lior, 22825 Kfar Rosh Hanikra (IL)
(74) Representative: Pearl Cohen Zedek Latzer Baratz UK LLP
(86) International application number: PCT/IL2012/050110
(87) International publication number: WO 2012/131679

(56) References cited:
- WO-A1-89/00151
- US-A- 4 040 963
- US-A- 5 185 079
- US-B1- 6 296 766

## Description

### FIELD

This invention relates to an apparatus for separating waste and uses thereof.

### BACKGROUND

Significant amounts of waste are generated on a daily basis by organizations and households, the majority of which is currently deposited in landfills. Apart from the decreasing amount of land available for such landfills, depositing waste in landfills causes many other problems as well, including pollution of water resources, objectionable odors and other impacts on the surrounding population. Furthermore, environmentally harmful green house gases (GHG) are produced in large quantities in landfills.

In order to overcome the environmentally harmful effect of GHG bioorganic waste can be converted into biogas, through a process that both reduces the volume and quantity of material to be deposited in landfills and also generates a new source of energy. Waste streams such as agriculture waste and household waste contain a high percentage of bioorganic matter. Anaerobic reactors can be used to convert the bioorganic matter into biogas. However, most of the waste stream contains other elements such as plastic, metal, glass, grit etc, which cannot be converted to biogas. Such non-bioorganic elements tend to physically block the reactor, interfere with the digestion process and contaminate output materials that could otherwise be used/reused, such as compostable organic residue for example.

Various methods and systems have been proposed to overcome this problem for biogas generation from waste streams. US Patent No. 5679263 describes an apparatus in which three different types of waste materials are fed into the system at three different points: liquid, solid and "sludgy" waste. However, non-bioorganic waste must be separated out before the bioorganic waste can be used for biogas generation. US Patent No. 4111798 relates to an apparatus for separating waste according to a selected density threshold: material that is less dense than a particular threshold floats while material that is denser than the threshold sinks, thereby allowing the two types of material to be separated. US Patent No. 2202772 relates to a digester which maintains sludge in a suspended state. US Patent No. 5185079 discloses an anerobic sequencing batch reactor. WO 89/00151 discloses a reactor for producing biogas. US Patent No. 6296766 discloses an anerobic digester system. US Patent No. 4040963 discloses an Anaerobic waste treatment facility.

### GENERAL DESCRIPTION

The present invention provides an apparatus according to claim 1. Such an apparatus comprises: a reactor for receiving waste material, said reactor having a top portion and a lower portion, wherein said reactor is filled with water up to the top portion of the reactor; a mixer for mixing said material with said water; a skimmer for removing a portion of said waste material having similar or lighter density than said water, said skimmer situated at the top portion of said reactor; and a settler for removing a portion of said waste material having higher density than said water, wherein said settler is situated at the lower portion of said reactor; and wherein said waste material comprises less than 10%wt of solids.

The term *"waste material"* should be understood to encompass any type of waste material (being non or partially sorted) from any source (being household, landfill, industrial, agricultural, marine, municipal, sewer, drain and so forth). Said waste material separated by an apparatus and process of the invention comprises organic materials from a biological source, non-bio organic material (i.e. organic material that isnot from a biological source, any organic synthetic material such as for example organic plastic material, organic polymeric material of any kind) and inorganic material from any source.

In an apparatus of the invention said waste material is mixed homogenously withwater in a reactor (in some embodiments equipped with a mixer). Said reactor having a higher top portion and a lower portion. During said mixing a portion of said waste material having density similar or lighter than the density of said water in said reactor (i.e. having the same density as said liquid or having density less than said water) is suspended at the top portion of said reactor and a portion of said waste material having density higher than the density of said water in said reactor settles to the lower portion of said reactor.

A skimmer (in some embodiments having a mechanical rotating arm) situated at the top portion of said reactor and is capable of receiving and skimming said waste material having density similar or lighter than the density of said water in said reactor toan outlet situated at said top portion of said reactor.

A settler (in some embodiments having a conical shape) situated at the lower portion of said reactor is capable of receiving said portion of said waste material having higher density than said water in said reactor and removing it through an outlet situated at said lower portion of said reactor.

In some embodiments said portion of said waste material having similar or similar or lighter density than said water comprises organic and non-bio organic waste material.

In other embodiments said portion of said waste material having higher density than said water comprises inorganic waste material.

In other embodiments an apparatus of the invention further comprises a diffuser for blowing gas bubbles into said reactor. In some embodiments said gas bubbles are micro-bubbles. In other embodiments, said gas is methane (in further embodiments said gas is biogas).

In further embodiments, said reactor further comprises anaerobic bacteria. Such anaerobic bacteria is capable of producing biogas from said waste material.

The waste material comprises less than 10%wt of solids.In embodiments said waste material comprises between about 2%wt - 8%wt of solids. When referring to weight percent of solids in said waste material it should be understood to relate to the weight amount of solids in said waste material that is not dissolved or hydrated prior to input into said apparatus of the invention. Since the solid weight in said waste material is low, hydrolyzation of said waste is performed with substantially no extraction or acetalyzation and/or methanolyzation of said waste.

In a further aspect the invention provides a process for separating waste material according to claim 9. Such processes comprise: inserting said waste material into an apparatus comprising a reactor for receiving waste material, said reactor having a top portion and a lower portion, wherein said reactor is filled with water up to the top portion of the reactor; mixing said waste material with water in said reactor using a mixer; removing a portion of said waste material having similar or lighter density than said water using a skimmer, wherein said skimmer is situated at the top portion of said reactor; and removing a portion of said waste material having higher density than said water using a settler, wherein said settler is situated at the lower portion of said reactor; and wherein said waste material comprises less than 10%wt of solids.

Further embodiments of a process of the invention comprises diffusing gas bubbles into said reactor.

In yet further embodiments of a process of the invention said reactor further comprises anaerobic bacteria. Thus, in some embodiments, a process of the invention further provides biogas from said waste material.

The waste material comprises less than 10%wt of solids. In other embodiments, said waste material comprises between about 2%wt - 8%wt of solids.

### BRIEF DESCRIPTION OF THE DRAWINGS

In order to understand the disclosure and to see how it may be carried out in practice, embodiments will now be described, by way of non-limiting example only, with reference to the accompanying drawings, in which:

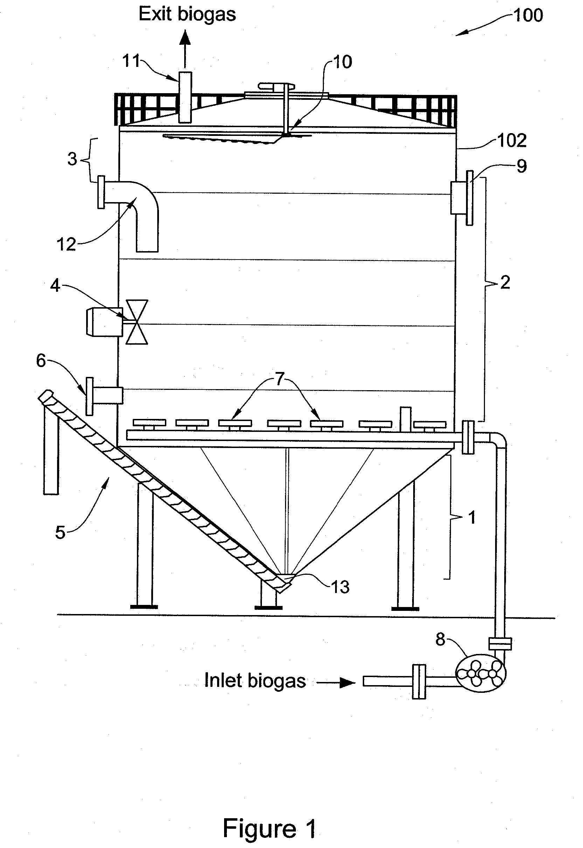

**Fig. 1** shows an exemplary, illustrative apparatus according to at least some embodiments of the present invention.

### DETAILED DESCRIPTION OF EMBODIMENTS

The present invention is directed to a separating reactor and method of its use for the efficient waste separation including the removal of non bioorganic matter as part of the process and, in some embodiments, generation of biogas from partially sorted waste.

The waste material is further characterized according to at least some embodiments of the present invention by optionally comprising at least about 40% organic waste, in other embodiments at least about 55%, in other embodiments at least about 65% organic waste, in further embodiments at least about 75% organic waste, in further embodiments at least about 85% organic waste and is some further embodiments at least about 95% organic waste.

As a non-limiting example, such waste may optionally be provided through a waste stream that is separated at the source and/or separated after collection, which may comprise also non-organic materials.

Said waste materials are separated in a reactor of the invention by use of a skimmer capable of removing similar or lighter (less dense) non-bioorganic materials, and a gravity dependent removal device to remove heavier (more dense) non-bioorganic materials.

The principles and operation of the present invention may be better understood with reference to the drawings and the accompanying description.

Referring now to the drawings, **Fig. 1** is of an exemplary, illustrative apparatus according to at least some embodiments of the present invention. According to this embodiment, the waste stream is preprocessed before entering the apparatus; however optionally the apparatus could itself perform the preprocessing (not shown). The waste stream has the previously described characteristics with regard to the percentage of organic material. The waste stream is also assumed to contain at least some solid elements.

For preprocessing, the waste stream is liquefied in a mixer. During the liquefaction process, previously processed effluent is added to the waste in the mixer. The mixed effluent/waste is pumped into the reactor of the system.

As shown, a system **100** features a reactor **102.** Reactor **102** comprises a settler **1,** a mixing volume **2** and a skimmer **3.** Said reactor is filled with water up to the top portion of the reactor. Settler **1** is formed from a conical lower section of reactor **102.** The middle section of reactor **102** is a cylinder for smoother mixing and reduced turbulence within mixing volume.

The waste stream is pumped through at least one entry **6,** of which only one is shown for the purpose of description only and without any intention of being limiting. A mechanical or hydraulic homogenizing mechanism (**4**) is applied to the reactor **102;** for example, homogenizing mechanism **4** may optionally be a simple mixer, whether mechanical or fluid based, but may also optionally feature other homogenizing components. The waste is mixed in the reactor **102** by homogenizing mechanism **4,** after which the heavy material that has a greater density (i.e. material having a specific gravity greater than the effluent) tends to drop down into the settler (**1**), due to gravity.

A mechanical element such as belt or screw conveyor (**5**) removes the heavy materials from the reactor **102,** which is connected to settler **1** through some type of separator and/or exit. As shown below, metals, glass and other heavy materials drop down to settler **1** and exit through a heavy materials exit **13** to conveyor **5.** Heavy materials exit **13** may optionally comprise a tube and/or a hole and/or a more elaborate separation apparatus, or a combination thereof.

Organic matter and light non-bioorganic materials such as plastic with similar specific densities remain in mixing volume **2** of reactor **102,** as their lower density prevents such materials from entering heavy materials exit **13.** To remove such non-bioorganic materials, a skimmer **3** removes these materials.

Optionally, according to at least some embodiments of the present invention, and in order to accelerate the further separation of bioorganic and non-bioorganic materials, fine bubble diffusers (**7**) are provided to reactor **102,** which in some embodiments attached in the lower section of the reactor **102,** in close proximity to but somewhat above the settler (1), so as to prevent the materials having density similar or lighter than water from entering settler **1,** while still maintaining circulation of the mixture of materials. In some embodiments processed biogas is optionally pumped to the diffusers **7** by a biogas blower (**8**). In other embodiments biogas from an external source (and/or another type of gas) could optionally be used. The bubbles are blown from the diffusers **7** and then move towards the top of the reactor **102** and hence towards skimmer **3.**

The bubbles blown from diffusers **7** are fine biogas bubbles, by which it is meant that the size of the bubbles is preferably from 10 to 100 microns and more preferably from 20 to 50 microns. As a non-limiting example only, the biogas bubbles may optionally be produced under a pressure of 100psi for example; the pressure is determined according to the height of reactor **102.** The use of such bubbles permits a higher separation rate and greater separation efficiency of reactor **102.** The bubbles are sized and pressured so as to flow upward with minimal turbulence or perturbations in the material within reactor **102.** Without wishing to be limited by theory, it is expected that these bubbles would nucleate on and cling to particle surfaces to increase the buoyant force of light materials such as plastics and bioorganic. Materials such as plastics that have a greater surface area would tend to capture more bubbles and hence would tend to float higher within reactor **102.**

Furthermore, injecting such bubbles into the fluid material in reactor **102** increases the overall amount of gas within reactor **102** and further enables phase separation between the different materials as described herein.

As noted above, it is stipulated that non-bioorganic material such as for example plastic material tends to capture the biogas bubbles; such bubbles reduce the overall specific weight of the material. The material therefore floats toward the top of the reactor **102** and hence toward skimmer **3,** floating on the top or upper surfaces of the effluent. An exit pipe (**9**) is located in the upper section of the reactor **102,** near skimmer **3.** The non-bioorganic and organic waste exits reactor **102** through this pipe **9.** A mechanical skimming element **10** such as a rotating skimming element or hydraulic flow assists the non-bioorganic material to flow in the exit direction and prevent it from blocking pipe **9,** if necessary optionally through the application of mechanical and/or fluid pressure. In some embodiments, the water (or general fluid or fluid/material mixture) level in reactor **102** is above pipe **9,** so as to prevent biogas generated or used during the separation process from escaping from reactor **102** through pipe **9.**

Reactor **102** may also produces biogas, optionally in addition to biogas provided through diffusers **7.** Biogas production for the purpose of this discussion is assumed to be an anaerobic process, although the present invention is not necessary limited to anaerobic biogas production. Such an anaerobic process may optionally comprise digestion and/or fermentation. Digestion is optionally performed by organisms, such as bacteria or yeast, which are known to be suitable for this task. For example, the process may optionally start with bacterial hydrolysis of the more complex organic materials to simpler sugars and amino acids, followed by conversion of the sugars and amino acids into carbon dioxide, hydrogen, ammonia, and organic acids by Acidogenic bacteria. Acetogenic bacteria then convert these resulting organic acids into acetic acid, along with additional ammonia, hydrogen, and carbon dioxide. Optionally, methanogens (methane producing bacteria) convert these products to methane and carbon dioxide, which is biogas; however reactor **102** may optionally not feature such methanogens.

In embodiments of the invention where biogas is produced in reactor **102,** it is removed through a biogas exit tube (**11**), which is located in the top of the reactor **102** and connected to the biogas blower (**8**) (the connection is not shown in the diagram), in order to provide the gas to the diffusers **7.** More specifically, biogas exit tube **11** is connected (through a tube or other connector(s) that are not shown) to an inlet of biogas blower **8** which is shown. A gas accumulator or other storage facility may also optionally be present in this circuit (not shown). Additionally or alternatively, some of the gas may be "bled off' or otherwise removed, for example for use as an energy source, and/or to maintain a preferred pressure (or pressure range) within the above described circuit and/or reactor **102.** Valves, pressure gauges and other components of such a circuit could easily be designed and implemented by one of ordinary skill in the art, in order to maintain the desired pressure(s), as different pressure(s) may optionally be implemented within different parts of the circuit (for example and without limitation, different pressures may optionally be implemented between reactor **102** and the remainder of the circuit).

Optionally the organic sludge or slurry (i.e. bio-organic material) may optionally be removed from reactor **102** through a bio-organic materials exit **12,** for example for recirculation and/or further processing (not shown). Bio-organic materials exit **12** may optionally comprise a tube and/or other connectors (not shown).

In addition, it is possible that plastic or heavy materials may be contaminated or mixed with desirable bio-organic material. Optionally, such mixtures are still removed as described above from reactor **102;** however, optionally such non bio-organic materials are reprocessed (for example optionally through post separation in a different unit or element such as for example magnet eddy current - not shown) in order to capture and separate additional bio-organic materials from such a mixture.

While the invention has been described with respect to a limited number of embodiments, it will be appreciated that many variations, modifications and other applications of the invention may be made.

## Claims

1. An apparatus (100) comprising:
a reactor (102) for receiving waste material, said reactor having a top portion and a lower portion, wherein said reactor is filled with water up to the top portion of the reactor;
a mixer (4) for mixing said material with said water;
a skimmer (3) for removing a portion of said waste material having similar or lighter density than said water, wherein said skimmer is situated at the top portion of said reactor; and
a settler (1) for removing a portion of said waste material having higher density than said water, wherein said settler is situated at the lower portion of said reactor; and
wherein said waste material comprises less than 10%wt of solids.

2. An apparatus according to claim 1, wherein said portion of said waste material having similar or lighter density than said water comprises organic and non-bio organic waste material.

3. An apparatus according to claim 1, wherein said portion of said waste material having higher density than said water comprises inorganic waste material.

4. An apparatus according to claim 1, further comprising a diffuser (7) for blowing gas bubbles into said reactor.

5. An apparatus according to claim 4, wherein said gas bubbles are micro-bubbles.

6. An apparatus according to claims 4 or 5, wherein said gas is selected from methane.

7. An apparatus according to any one of the preceding claims, wherein said reactor further comprises anaerobic bacteria.

8. An apparatus according to any one of the preceding claims, wherein said waste material comprises between about 2%wt- 8%wt of solids.

9. A process for separating waste material comprising: inserting said waste material into an apparatus (100) comprising a reactor (102) for receiving waste material, said reactor having a top portion and a lower portion; wherein said reactor is filled with water up to the top portion of the reactor; mixing said waste material with said water in said reactor using a mixer (4), removing a portion of said waste material having similar or lighter density than said water using a skimmer (3), wherein said skimmer is situated at the top portion of said reactor; and removing a portion of said waste material having higher density than said water using a settler (1), wherein said settler is situated at the lower portion of said reactor; and wherein said waste material comprises less than 10%wt of solids.

10. A process according to claim 9, further comprising diffusing gas bubbles into said reactor.

11. A process according to claim 10, wherein said gas bubbles are micro-bubbles.

12. A process according to claims 9 or 10, wherein said gas is selected from methane.

13. A process according to any one of claims 9 - 12, wherein said reactor further comprises anaerobic bacteria.

14. A process according to claim 13, further producing biogas from said waste material.

## Patentansprüche

1. Vorrichtung (100), umfassend:
einen Reaktor (102) zum Aufnehmen von Abfallmaterial, wobei der Reaktor einen oberen Abschnitt und einen unteren Abschnitt aufweist, wobei der Reaktor bis zum oberen Abschnitt des Reaktors mit Wasser gefüllt ist;
einen Mischer (4) zum Mischen des Materials mit dem Wasser;
einen Skimmer (3) zum Entfernen eines Teils des Abfallmaterials mit einer ähnlichen oder geringeren Dichte als das Wasser, wobei sich der Skimmer in dem oberen Abschnitt des Reaktors befindet; und
einen Absetzer (1) zum Entfernen eines Teils des Abfallmaterials mit höherer Dichte als das Wasser, wobei sich der Absetzer in dem unteren Teil des Reaktors befindet; und
wobei das Abfallmaterial weniger als 10 Gew.-% Feststoffe umfasst.

2. Vorrichtung nach Anspruch 1, wobei der Teil des Abfallmaterials mit einer ähnlichen oder geringeren Dichte als das Wasser organisches und nicht-bioorganisches Abfallmaterial umfasst.

3. Vorrichtung nach Anspruch 1, wobei der Teil des Abfallmaterials mit einer höheren Dichte als das Wasser anorganisches Abfallmaterial umfasst.

4. Vorrichtung nach Anspruch 1, ferner umfassend einen Diffusor (7) zum Einblasen von Gasblasen in den Reaktor.

5. Vorrichtung nach Anspruch 4, wobei die Gasblasen Mikroblasen sind.

6. Vorrichtung nach Anspruch 4 oder 5, wobei das Gas aus Methan ausgewählt ist.

7. Vorrichtung nach einem der vorhergehenden Ansprüche, wobei der Reaktor ferner anaerobe Bakterien umfasst.

8. Vorrichtung nach einem der vorhergehenden Ansprüche, wobei das Abfallmaterial zwischen etwa 2 Gew.-% und 8 Gew.-% Feststoffe umfasst.

9. Verfahren zum Trennen von Abfallmaterial, umfassend: Einführen des Abfallmaterials in eine Vorrichtung (100), die einen Reaktor (102) zum Aufnehmen von Abfallmaterial umfasst, wobei der Reaktor einen oberen Abschnitt und einen unteren Abschnitt aufweist; wobei der Reaktor bis zum oberen Abschnitt des Reaktors mit Wasser gefüllt ist; Mischen des Materials mit dem Wasser in dem Reaktor unter Verwendung eines Mischers (4), Entfernen eines Teils des Abfallmaterials mit einer ähnlichen oder geringeren Dichte als das Wasser unter Verwendung eines Skimmers (3), wobei sich der Skimmer in dem oberen Abschnitt des Reaktors befindet; und Entfernen eines Teils des Abfallmaterials mit höherer Dichte als das Wasser unter Verwendung eines Absetzers (1), wobei sich der Absetzer in dem unteren Teil des Reaktors befindet; und wobei das Abfallmaterial weniger als 10 Gew.-% Feststoffe umfasst.

10. Verfahren nach Anspruch 9, ferner umfassend Diffundieren von Gasblasen in den Reaktor.

11. Verfahren nach Anspruch 10, wobei die Gasblasen Mikroblasen sind.

12. Verfahren nach Anspruch 9 oder 10, wobei das Gas aus Methan ausgewählt ist.

13. Verfahren nach einem der Ansprüche 9 - 12, wobei der Reaktor ferner anaerobe Bakterien umfasst.

14. Verfahren nach Anspruch 13, ferner erzeugend Biogas aus dem Abfallmaterial.

## Revendications

1. Appareil (100) comprenant :
un réacteur (102) pour recevoir des déchets, ledit réacteur ayant une partie supérieure et une partie inférieure, ledit réacteur étant rempli d'eau jusqu'à la partie supérieure du réacteur ;
un mélangeur (4) pour mélanger ledit matériau avec ladite eau ;
un skimmer (3) pour retirer une partie desdits déchets ayant une densité similaire ou moins élevée que celle de ladite eau, ledit skimmer étant situé au niveau de la partie supérieure dudit réacteur ; et
un décanteur (1) pour éliminer une partie desdits déchets ayant une densité plus élevée que ladite eau, ledit décanteur étant situé au niveau de la partie inférieure dudit réacteur ; et
dans lequel lesdits déchets comprennent moins de 10 % en poids de solides.

2. Appareil selon la revendication 1, dans lequel ladite partie desdits déchets ayant une densité similaire ou inférieure à celle de ladite eau comprend des déchets organiques et non bio-organiques.

3. Appareil selon la revendication 1, dans lequel ladite partie desdits déchets ayant une densité plus élevée que ladite eau comprend des déchets inorganiques.

4. Appareil selon la revendication 1, comprenant en outre un diffuseur (7) pour insuffler des bulles de gaz dans ledit réacteur.

5. Appareil selon la revendication 4, dans lequel lesdites bulles de gaz sont des microbulles.

6. Appareil selon les revendications 4 ou 5, dans lequel ledit gaz est choisi parmi le méthane.

7. Appareil selon l'une quelconque des revendications précédentes, dans lequel ledit réacteur comprend en outre des bactéries anaérobies.

8. Appareil selon l'une quelconque des revendications précédentes, dans lequel lesdits déchets comprennent entre environ 2 % en poids et 8 % en poids de solides.

9. Procédé de séparation de déchets consistant à :
introduire lesdits déchets dans un appareil (100) comprenant un réacteur (102) pour recevoir des déchets, ledit réacteur ayant une partie supérieure et une partie inférieure ;
dans lequel ledit réacteur est rempli d'eau jusqu'à la partie supérieure du réacteur ;
mélanger lesdits déchets avec ladite eau dans ledit réacteur au moyen d'un mélangeur (4) ;
retirer une partie desdits déchets ayant une densité similaire ou moins élevée que ladite eau au moyen d'un skimmer (3) ;
dans lequel ledit skimmer est situé au niveau de la partie supérieure dudit réacteur ; et
retirer une partie desdits déchets ayant une densité plus élevée que ladite eau au moyen d'un décanteur (1) ;
dans lequel ledit décanteur est situé au niveau de la partie inférieure dudit réacteur ; et
dans lequel lesdits déchets comprennent moins de 10 % en poids de solides.

10. Procédé selon la revendication 9, comprenant en outre la diffusion de bulles de gaz dans ledit réacteur.

11. Procédé selon la revendication 10, dans lequel lesdites bulles de gaz sont des microbulles.

12. Procédé selon les revendications 9 ou 10, dans lequel ledit gaz est choisi parmi le méthane.

13. Procédé selon l'une quelconque des revendications 9 à 12, dans lequel ledit réacteur comprend en outre des bactéries anaérobies.

14. Procédé selon la revendication 13, produisant en outre du biogaz à partir desdits déchets.
